# EUROPEAN PATENT APPLICATION

(11) **EP 4 741 824 A1**
(43) Date of publication of application: **13.05.2026**
(21) Application number: 25194302.3
(22) Date of filing: 06.08.2025
(51) Int. Cl.: G01N 33/483

(54) **SYSTEM AND METHOD FOR LONGITUDINAL ANALYSIS OF AN ORGANOID**

(30) Priority: 07.11.2024 US 202418940699
(71) Applicant: Harvard Bioscience, Inc., Holliston, MA 01746 (US)
(72) Inventor: COLLAZO, Julio Alvarez, Norderstedt (DE); GREEN, James Walter, BOSTON (US); MEENTS, Jannis Enno, KUSTERDINGEN (DE); SAVARD, Serge, BOSTON (US); Schönecker, Sven-Mark, Nürtingen (DE); Shetty, Nityanand, Hopkinton (US)
(74) Representative: Gevers Patents

(57) **Abstract**

A system (102) for longitudinal analysis of an organoid includes a region (106) configured to support a Microelectrode Array (MEA) device (108) carrying an organoid for longitudinal analysis. The MEA device (108) includes a MEA supporting the organoid, a well configured to hold perfusion media for the organoid, a port for supplying the perfusion media to the well, and nodes in the MEA for sensing electrophysiological (EP) signals produced by the organoid from an interior of the organoid. The system (102) further includes a perfusion media interface to a perfusion system (114). The system (102) further includes a computing system (126) configured to identify events in the EP signals for analysis based on predetermined criteria. The identified events include less than an entirety of each of the EP signals, provide a summary of the identified events, and analyze a user selected set of the identified events in the summary.

## Description

### TECHNICAL FIELD

The following generally relates to analysis of an organoid, and finds particular application to a system and method for longitudinal analysis of an organoid.

### BACKGROUND

An organoid includes a collection of cells of a specific type (e.g., brain, heart, etc.) grown from a few cells of the tissue itself or stem cells that differentiate into cells of the specific organ and behave similarly to the specific organ. In general, it can be considered a smaller version of the specific organ that exhibits many of the functional, structural, and/or biological complexity of the specific organ. As such, an organoid can be utilized to help understand organ behavior and/or how the organ responds to a stimulus (e.g., electrical, optical, thermal, etc.) and/or a compound under test (CUT) (e.g., a drug or other therapy being evaluated in therapy discovery or safety and toxicology testing applications in therapy development) over time. This has been achieved through *in vitro* studies in which an organoid is grown on a Microelectrode Array (MEA) and organoid electrical activity, in an absence and/or presence of a stimulus and/or a CUT, is measured through microelectrodes of the MEA, where the electrical activity is indicative of behavior and behavior in response to the stimulus and/or the CUT.

In general, an MEA device includes an MEA for growing an organoid, a reservoir for holding perfusion media (which promotes growth of the organoid and provides nourishment to maintain viability of the organoid) and a CUT, a port(s) to supply the perfusion media and the CUT to the reservoir, and electrodes for routing electrophysiological (EP) signals produced by the organoid before, during and/or after a CUT, a stimulus, etc. to an analysis system. The perfusion media and/or CUT is supplied to the reservoir of the mesh MEA using a pump and fluidics or manually. With pump-based perfusion media and CUT approach, the user would program the pumps to supply the perfusion media and CUT to the reservoir, each based on its own schedule. With a manual approach, the user would utilize a pipette or the like to supply the perfusion media and CUT to the reservoir, each based on its own schedule.

Non-limiting examples of a mesh MEA device are discussed in Stumpp et al., "Scalable mesh microelectrode arrays for neural spheroids and organoids," Current Directions in Biomedical Engineering, vol. 9, no. 1, 2023, pp. 575-578. https://doi.org/10.1515/cdbme-2023-1144, and McDonald et al. "A mesh microelectrode array for non-invasive electrophysiology within neural organoids," Biosens Bioelectron. 2023 May 15;228:115223. doi: 10.1016/j.bios.2023.115223. Epub 2023 Mar 11. PMID: 36931193. The mesh MEA device of Stumpp et al. is compatible with 60-channel amplifiers and includes a well supporting a polyimide grid mesh containing traces and microelectrodes at nodes, and access ports to fill the volume below the mesh with perfusion media and the CUT. The mesh MEA device of McDonald et al. is compatible with 256-channel amplifiers and includes a well supporting a central "spider-web-like" mesh containing microelectrodes sparsely distributed across the region, and access ports to fill the volume below the mesh with perfusion media and the CUT.

The analysis system stores, displays, and provides tools for analyzing the EP signals. An example analysis includes analyzing the EP signals of a perfused organoid where no CUT has been supplied to establish a baseline signal. This may be performed without a stimulus where the organoid naturally produces EP signals or with a stimulus, e.g., where the organoid produces EP signals in response to a stimulus. Another example analysis includes analyzing the EP signals over time to monitor the growth of the organoid, where more microelectrodes of the mesh MEA will provide measurable signals over time as the organoid grows over them. Another example analysis includes analyzing the EP signals after supplying a CUT and comparing the EP signals with the baseline. Another example analysis includes analyzing the EP signals after supplying a compound that produces a predictable response to determine.

For the analysis, snippets of measured EP signals for a given time duration are graphically displayed via individual windows, each window corresponding to a microelectrode. The user would observe the snippets and analyze a portion of any EP signal determined to be of interest. By way of a non-limiting example, for a 60-microelectrode MEA, the display would include sixty windows, each corresponding to a microelectrode. Each window would display a snippet of the measured signal for the time duration of the window. For an hour-long study and a window time duration of one minute, the user would have to scroll through at least sixty sets of windows to observe the entirety of each of the measured EP signals.

For longer studies, e.g., studies over a day, a month, a year, etc., the volume of the measured data (i.e., the EP signals) may be on an order of terabytes such that the above-discussed analysis thereof may not be feasible or practical within a reasonable amount of time for the study. In addition, the user would be tasked with monitoring the supply of perfusion media and CUT to ensure the scheduled rates and frequency for the perfusion media to promote the desired growth and maintain viability, and the scheduled rates and frequency of the CUT satisfy the goals of the study. For example, the user may need to adjust the schedule and reprogram a pump based on the monitoring, a study may need to be repeated where too much or too little perfusion media was supplied, and the organoid functions abnormally or dies or too much or too little CUT is supplied to provide data to evaluate the initiatives set forth in the study.

In view of at least the foregoing, there is an unresolved need for an improved system and/or method for longitudinal analysis of an organoid.

### SUMMARY

Aspects described herein address the above-referenced problems and/or others.

In one aspect, a system for longitudinal analysis of an organoid includes a region configured to support a Microelectrode Array (MEA) device carrying an organoid for longitudinal analysis. The MEA device includes a MEA supporting the organoid, a well configured to hold perfusion media for the organoid, a port for supplying the perfusion media to the well, and nodes in the MEA for sensing electrophysiological (EP) signals produced by the organoid from an interior of the organoid. The system further includes a perfusion media interface to a perfusion system. The system further includes a computing system configured to identify events in the EP signals for analysis based on predetermined criteria. The identified events include less than an entirety of each of the EP signals, provide a summary of the identified events, and analyze a user selected set of the identified events in the summary.

In another aspect, a method for longitudinal analysis of an organoid includes introducing an organoid to a MEA device. The method further includes supplying perfusion media to the MEA device to maintain organoid viability for the longitudinal analysis. The method further includes sensing EP signals produced by the organoid from an inside of the organoid with nodes of the MEA device. The method further includes processing the EP signals with a computing system. The processing includes identifying events for analysis in the EP signals based on predetermined criteria, providing a summary of the identified events, and analyzing a user selected set of the identified events in the summary.

Those skilled in the art will recognize still other aspects of the present application upon reading and understanding the attached description.

### BRIEF DESCRIPTION OF THE DRAWINGS

The invention may take form in various components and arrangements of components, and in various steps and arrangements of steps. The drawings are only for the purposes of illustrating the embodiments and are not to be construed as limiting the invention.
FIG. 1 schematically illustrates an example of a system configured for longitudinal analysis of *in vitro* organoid studies, in accordance with an embodiment(s) herein.
FIG. 2 illustrates an example of operating modes of the system for longitudinal analysis of *in vitro* organoid studies, in accordance with an embodiment(s) herein.
FIG. 3 illustrates an example of a data analysis module of the system for longitudinal analysis of *in vitro* organoid studies, in accordance with an embodiment(s) herein.
FIG. 4 schematically illustrates a variation of the system described in connection with FIGURE 1.
FIGS. 5-9 illustrate examples of events of interest in neurological signals, in accordance with an embodiment(s) herein.
FIGS. 10-12 illustrate examples of events of interest in cardiac signals, in accordance with an embodiment(s) herein.
FIG. 13 illustrates an example method for longitudinal analysis of *in vitro* organoid studies, in accordance with an embodiment(s) herein.
FIG. 14 illustrates another example method for longitudinal analysis of *in vitro* organoid studies, in accordance with an embodiment(s) herein.
FIG. 15 illustrates yet another example method for longitudinal analysis of *in vitro* organoid studies, in accordance with an embodiment(s) herein.

### DESCRIPTION OF EMBODIMENTS

The following describes systems and methods for longitudinal analysis of *in vitro* organoid studies, including organoids such as neural organoids, cardiac organoids, hepatic organoids, muscular organoids, and/or other organoids. The systems and methods, in one instance, includes automated evaluation of the acquired EP signals by a computing system to identify snippets of interest therein for subsequent analysis. The snippets are identified based on predetermined criteria and presented to a user via a summary, where the user can select one or more of the snippets in the summary for analysis. In some instances, the system and/or method include automated control of the perfusion media and/or CUT based at least on the acquired EP signals (and in some instances further on perfusion media and/or CUT feedback and/or perfusion media and/or CUT system settings, etc.).

The identification of snippets of interest and the summary thereof with user selectable links (e.g., a hyperlink, etc.) to the snippets can facilitate analyzing large volumes of acquired EP signals for longitudinal analysis of organoids in a reasonable amount of time where, without the approach described herein, it may be not feasible or practical to analyze the large volume of acquired EP signals within a reasonable amount of time for the study, e.g., due to acquiring terabytes of EP signals each day. Automated control of the perfusion media and/or CUT based at least on the acquired EP signals can facilitate supplying perfusion media and/or CUT respectively to promote growth and maintain viability and satisfy initiatives of the study, with or without additional monitor by the user.

Analysis (human and/or machine) of the snippets can be utilized to streamline discovery, evaluation, safety testing, toxicity testing, and/or efficacy testing of pharmaceuticals or other therapies. The approach described herein can facilitate longer-term longitudinal studies (e.g., >30 days) to determine response of an organoid to a compound under test and/or reduce the need for manual or operator-initiated perfusion and/or application of the compound under test. In addition, the approach described herein can provide improved data analysis in organoid testing, for example by assisting the user in identifying information indicative of the functioning (or abnormalities in the functioning) of an organoid in response to a compound under test. Some aspects have particular application to the *in vitro* testing of organoids in neurological and cardiac applications.

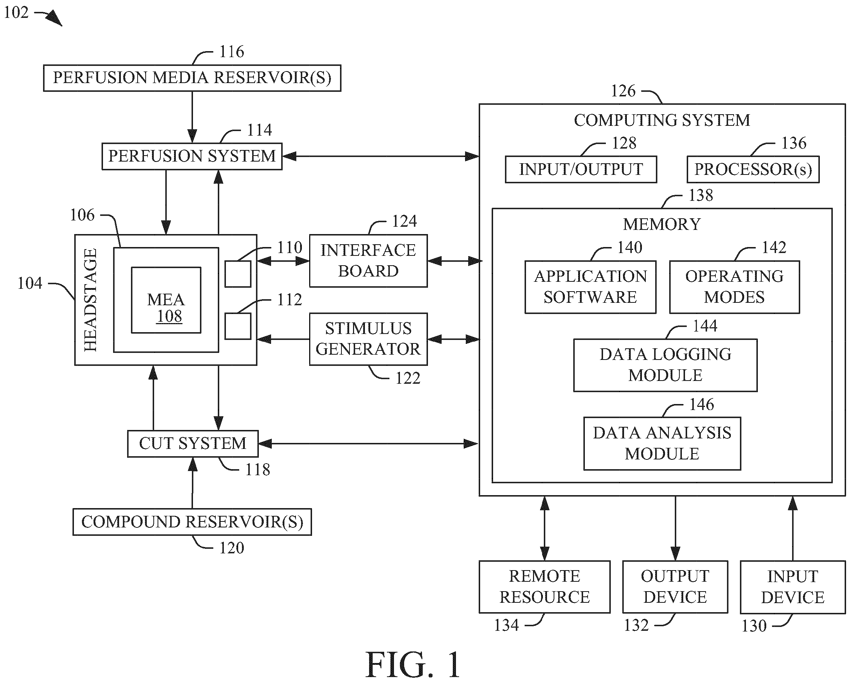

Initially referring to FIG. 1, a system 102 for longitudinal analysis of organoids is schematically illustrated. The system 102 includes a headstage 104 with one or more MEA device receiving regions 106, each configured to receive an MEA device 108 for longitudinal analysis. Examples of suitable MEA devices 108 include two-dimensional (2-D) and/or three-dimensional (3-D), mesh and/or non-mesh MEA devices. While the following description is described primarily with reference to mesh MEA devices, it will be recognized that other MEA structures, such as flexible MEAs, perforated MEAs or permeable MEA-membranes may also be used. Preferably, however, the MEA has a relatively flexible, 3-D structure which minimizes deformation of the organoid and receives EP signals from a plurality of locations within the organoid (e.g., sense EP signals produced by the organoid from an interior of the organoid, from an inside of the organoid, etc.). The headstage 104 further includes components 110 such as electrodes configured to electrically communicate with microelectrodes of the mesh MEA device 108 to receive signals (e.g., produced by an organoid) from the mesh MEA and/or provide signals (e.g., stimulus, etc.) to the MEA.

In one instance, the components 110 further include one or more amplifiers configured to amplify the EP signals, one or more analog-to-digital converters configured to convert analog EP signals to digital EP signals, a multiplexor to route the EP signals, fluidics to supply perfusion media and/or CUT from an external reservoir/source to the MEA device 108 in the one or more MEA device receiving regions 106, etc. In some instances, the components 110 further include components for providing perfusion media feedback and/or CUT feedback, such as composition, concentration, temperature, pH, etc. in connection with the perfusion media and/or CUT. The headstage 104 further includes an interface 112 (e.g., wireless and/or wired) for routing EP signals off the headstage 104 and/or external signals to the headstage 104.

The headstage 104 may be based on and/or include the MEA2100 headstage, the MEA2100-Mini-System headstage, the MCS MEA2100-Beta-Screen headstage, the MCS Multiwell-MEA headstage, all products of Multi Channel Systems MCS GmbH, DE. Other headstages are also contemplated herein. In another instance, the headstage 104 does not include at least one of an amplifier, an analog-to-digital converters, and/or other processing component. In such a configuration, another component(s), e.g., a separate amplifier, a separate analog-to-digital converter, and/or a separate processing component is utilized to perform the corresponding function.

An example mesh MEA device 108 includes a well with a mesh MEA configured to carry an organoid, a reservoir for perfusion media to support organoid perfusion over study period and a CUT, an access port(s) for receiving the perfusion media and the CUT, and an electrical interface for routing EP signals generated by the organoid off the mesh MEA device 108. A single reservoir would include a mixture of both perfusion media and a CUT (when used). This allows for concurrent feeding and testing of the organoid. Multiple reservoirs allow for a mixture of both in one reservoir and only perfusion media in another reservoir. This may facilitate difference measurements between an organoid supplied only with perfusion media and the organoid supplied with the mixture of perfusion media and the CUT. Examples of suitable mesh MEAs are discussed herein.

The system 102 further includes a perfusion system 114. The perfusion system 114 is configured to provide perfusion media for the organoid of the MEA device 108. The perfusion system 114 includes one or more reservoirs 116 for holding one or more supplies of perfusion media. The perfusion system 114 further includes a pump system for supplying the perfusion media from the one or more reservoirs 116 to the mesh MEA device 108. In one instance, the perfusion system 114 is programmable with settings to vary the concentration, composition, and/or other characteristics of the perfusion media according to desired setpoints. In this instance, the perfusion system 114 is operated either under human control or computer control, automatically or semiautomatically. In some instances, such control includes control of multiple perfusion media from multiple reservoirs 116, the proportions of which are changed over the course of the study.

In some instance, the perfusion system 114 is configured to receive the perfusion media feedback, such as composition, concentration, temperature, pH, etc., where the components 110 of the headstage 104 provide such data. In such instances, the perfusion system 114 can automatically or semi-automatically (e.g., under operator control) control the concentration, the composition, and/or other characteristic of the perfusion media based on the feedback. In some instance, the perfusion system 114 is further configured to provide information such as the feedback from the MEA device 108, the setpoints, warnings, error codes, etc., for analysis by a computing system (described in greater detail below), which may provide control signals to the perfusion system 114 based thereon and/or in connection with CUT and/or EP signal information, and the perfusion system 114 can automatically or semi-automatically (e.g., under operator control) control the concentration, the composition, and/or other characteristic of the perfusion media based on the control signals.

The system 102 further includes a CUT system 118. The CUT system 118 is configured to provide a CUT for the organoid of the MEA device 108. The CUT system 118 includes one or more reservoirs 120 for holding one or more supplies of CUT. The CUT system 118 further includes fluidics and a pump system for supplying the CUT from the one or more reservoirs 120 to the mesh MEA device 108. In one instance, the CUT system 118 is programmable with settings to vary the concentration, composition, and/or other characteristics of the CUT according to desired setpoints. In this instance, the CUT system 118 is operated either under human control or computer control, automatically or semiautomatically. In some instances, such control includes control of multiple CUTs from multiple reservoirs 120, the proportions of which are changed over the course of the study.

In some instance, the CUT system 118 is configured to receive the CUT feedback, such as composition, concentration, temperature, pH, etc., where the components 110 of the headstage 104 provide such data. In such instances, the CUT system 118 can automatically or semi-automatically (e.g., under operator control) control the concentration, the composition, and/or other characteristics of the CUT based on the feedback. In some instance, the CUT system 118 is further configured to provide information such as the feedback from the MEA device 108, the setpoints, warnings, error codes, etc., for analysis by a computing system (described in greater detail below), which may provide control signals to the CUT system 118 based thereon and/or in connection with perfusion media and/or EP signal information, and the CUT system 118 can automatically or semi-automatically (e.g., under operator control) control the concentration, the composition, and/or other characteristic of the perfusion media based on the control signals.

In some instances, the system 102 further includes a stimulus generator 122. The stimulus generator 122 is configured to provide at least one stimulus for the organoid. Examples of suitable stimuli include optical (e.g., visible light, etc.), electromagnetic (electric or magnetic), thermal (heating and/or cooling), chemical, mechanical (e.g., vibration, etc.), etc. For electrical stimulation, the stimulus generator 122 supplies an electrical stimulus using the microelectrodes of the mesh MEA device 108 and/or other electrodes. In one instance, the stimulus generator 122 is programmable with settings that indicate a type of stimulus, a pattern (e.g., a sine wave, a ramp function, a rectangular pulse, etc.) for the stimulus, upload a biological signal to be used as a stimulation pattern, etc. In this instance, the stimulus generator 122 is operated either under human control or computer control, automatically or semiautomatically. An example of a suitable electrical (voltage and/or current) stimulus generator includes the STG5 Stimulus Generator, a product of Multi Channel Systems MCS GmbH, DE. Other stimulus generators are also contemplated herein.

The system 102 further includes an interface 124. In general, the interface 124 serves as a bridge between the headstage 104 and a data analyzer. For example, the interface 124 includes an input port for receiving EP signals from the headstage 104, components such as one or more digital-to-analog converters (DACs), one or more analog-to-digital converters (ADCs), one or more auxiliary input and/or output ports, one or more multiplexors, and/or other component(s), and an output port for transferring the received and/or further processed EP signals off the interface 124. An example of a suitable interface 124 includes the interface board multiboot (IFB-C), a product of Multi Channel Systems MCS GmbH, DE. Other interface boards are also contemplated herein.

The system 102 further includes a computing system 126. The computing system 126 may include a computer, a workstation, a server, cloud services, distributed computing services, etc. The computing system 126 includes input/output (I/O) 128. An input device(s) 130 includes a keyboard, mouse, touchscreen, microphone, etc. An output device 132 includes a human readable device such as a display monitor or the like. A remote resource 134 includes another computing system, a database, a server, cloud storage and/or computing, including resources distributed over multiple locations / data centers, etc. The input device 130, the output device 132, and/or the remote resource 134 are in electrical communication with the computing system 126 via the I/O 128 and/or otherwise.

The computing system 126 further includes at least one processor 136 such as a microprocessor (µP), a central processing unit (CPU), graphics processing unit (GPU), etc., and a computer readable medium 138 ("MEMORY"), which includes non-transitory medium and excludes transitory medium (signals, carrier waves, and the like). The computer readable medium 138 at least includes application software 140, operating modes 142, a data logging module 144, and a data analysis module 146. In another example, the data analysis module 146 is part of a different computing system that is separate from the computing system 102. In one instance, such a separate system is configured to provide retrospective analysis and/or review of the data provided by the data logging module 144.

The application software 140 presents a user interface on the output device 132. The user interface allows a user to set up and/or change parameters of a study such as program and/or control the perfusion system 114, the CUT system 118, the stimulus generator 122, the interface board 124, etc., via the input device 130. In one instance, this includes programming the perfusion system 114 and the CUT system 118 with settings to vary the concentration, composition, and/or other characteristics of the perfusion media and/or CUT according to corresponding setpoints. In another instance, this includes programming the stimulus generator 122 to select an electrode of the mesh MEA device 108 for stimulation, set a stimulus pattern, etc.

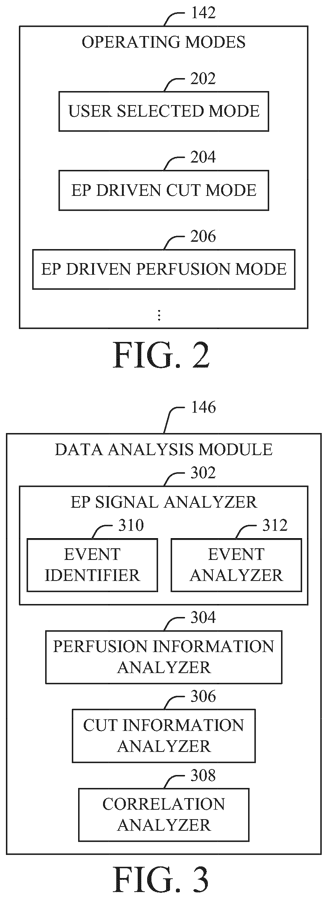

The application software 140 further allows the user to activate an operating mode of the operating modes 142. Examples of operating modes 142 are shown in FIG. 2 and include a user selected mode 202, an EP driven CUT mode 204, an EP driven perfusion mode 206, and/or other mode(s). The user selected mode 202 applies CUT and perfusion media according to a schedule and/or amounts selected by user. The EP driven CUT mode 204 applies CUT as a function of the EP signals. In one example, this is based on desired spatial and/or temporal values. By way of example, the EP driven CUT mode applies CUT if the EP signals fall below a predetermined threshold value. The EP driven perfusion mode 206 applies perfusion as a function of the EP signals.

Returning to FIG. 1, the data logging module 144 is configured to receive the EP signals over time from the interface 124 and record the EP signals, e.g., in the memory 138, the remote resource 134, and/or elsewhere. For a 2-D mesh MEA, the logging module 144 records the EP signals in 3-D such as x and y coordinate location of electrode of the mesh MEA device 108 and a time stamp. For a 3-D mesh MEA device 108, the data logging module 144 records the EP signals in four-dimensions (4-D) such as x, y and z coordinate location of electrode of the mesh MEA device 108 and a time stamp. In some instances, the data analysis module 146 is further configured to receive the information provided by the perfusion system 114 and/or the information provided by the CUT system 118, including the feedback from the MEA device 108, the setpoints, warnings, error codes, etc., and record the information in the memory 138, the remote resource 134, and/or elsewhere. The logging module 144 correlates the recorded EP signals, perfusion information, and CUT information for review and analysis.

The data analysis module 146 is configured to analyze the logged data, including at least the EP signals, and, where recorded, the information provided by the perfusion system 114 and/or the information provided by the CUT system 118. FIG. 3 illustrates an example of the data analysis module 146 that includes an EP signal analyzer 302, a perfusion information analyzer 304, a CUT information analyzer 306, and a correlation analyzer 308. The EP signal analyzer 302 includes an event identifier 310 and an event - driven analyzer 312.

The event identifier 310 analyzes the recorded EP signals and identifies signals of the recorded EP signals that are likely to be of interest to the user. The identification can be based on default, user defined, artificial intelligence (AI) generated, etc. analysis criteria. In one instance, the analysis includes one or more of analyzing an EP signal before, during and/or after supplying a CUT to the organoid based on the analysis criteria, comparing EP signals from different electrodes of the mesh MEA device 108 within a same region of interest and/or different regions of interest based on the analysis criteria, different time regions of a same EP signal, etc. The processing may further include comparing EP signals to predetermined thresholds, etc.

By way of non-limiting example, the event identifier 310 may apply a threshold or other criteria to determine whether an EP signal, after a CUT is supplied, represents an expected response or an "abnormal" response that should be identified for subsequent analysis by the user. To facilitate the analysis the EP signal analyzer 302 may process the EP signals to determine one or more of a minimum amplitude, a maximum amplitude, a time of minimum, a time of maximum, a peak-to-peak amplitude, a mean value, a variance, a distribution, a slope, a duration, a width, a shape, an area under a curve, a ratio between snippets of EP signals, a frequency or range thereof, a number of spikes, etc., to filter the recorded EP signals, to perform spatial, temporal, and/or frequency domain analysis, etc.

The event identifier 310, for parts of EP signals that satisfy the analysis criteria, identifies the parts of EP signals as events. In one instance, this may include creating a summary that includes, for each event, an electrode identification, a date, a time or time range, an x,y or x,y,z location, etc. In one instance, the summary information is presented to the user in an on-screen widget such as a list box, a drop-down menu, a combo box, a check box, etc. The user can then select one or more of the identified events, individually or in a batch, to visually present for analysis, along with the electrode identification, a date, a time or time range, an x,y or x,y,z location, etc. In one instance, the criteria that was satisfied is also provided to the user.

In another instance, the summary information is presented to the user as individual graphs of the events. Similarly, the user can then select one or more of the identified events, individually or in a batch, to visually present for analysis. Information such as the electrode identification, a date, a time or time range, an x,y or x,y,z location, etc., can also be provided. For instance, hovering an input device such as a mouse pointer over a graph can invoke a pop-up window displaying such information for the snippet in the graph. Likewise, the criteria that were satisfied can also be provided to the user. For example, if the satisfied criteria included a change in amplitude (outside of the noise floor), the information may include the amplitude before the change, the amplitude at the change, the amplitude after the change, etc.

Again, the user can select events included in the summary to analyze. The user can also select parts of an EP signal not included in the summary. In either or both instances, the selected snippets can be visually presented to the user, e.g., through a graphical representation. An example of suitable graphical representation is provided by the Multi Channel Analyzer platform, a product of Multi-Channel Systems MCS GmbH, DE. By way of non-limiting example, the graphical representation may include a display of multiple windows, including a different window for one or more of the electrodes, with a snippet for an identified event in a window. Where there are multiple windows, selecting a window would enable processing tools for analyzing the snippet in the window.

The event analyzer 312 analyzes events identified by the event identifier 310. For example, in one instance the event analyzer 312 provides information on the number and spatial distribution of the identified events, as well as their temporal relationship to the concentration of the CUT. The analysis may be presented, for example, by way of a histogram that represents the number of an event or event of interest as a function of time. In another example, the event analyzer 312 provides information of the spatial distribution of identified events. In still another example, the event analyzer may provide information indicative of the mean, rate of change, or the rate of change of the rate of change of the signals generated by locations within the organoid. Signals from portions of the organoid may also be masked or discarded so as not to analyze signals from areas that are known to be necrotic or otherwise of a type identified by the event identifier 310.

The event analyzer 312 can provide the same or similar processing utilized by the event identifier 310. For example, the event analyzer 312 provides the user with tools for determining one or more of a minimum amplitude, a maximum amplitude, a time of minimum, a time of maximum, a peak-to-peak amplitude, a mean value, a variance, a distribution, a slope, a duration, a width, a shape, an area under a curve, a ratio between snippets of EP signals, frequency or frequency range, a number of spikes, etc., filtering the snippets, performing spatial, temporal, and/or frequency domain analysis on the snippets, etc. The snippets and/or processed snippets and/or other information can be visually presented as individual events, side-by-side comparisons, overlaid comparisons, an average across electrodes, a distribution over the mesh MEA microelectrode array, etc.

Example filters include a 0^{th} order filter, 1^{st} order filter, 2-D filter, and/or other filter. One or more of these filters can be implemented using convolution operations with specific kernels or masks. For example, an averaging filter might use a simple mean kernel, while edge detection filters use more complex kernels to highlight changes in the EP signal. A 0^{th} order filter can be utilized to smooth the EP signal by averaging values of neighboring data points. In one instance, this may reduce noise and/or highlight an overall trend of the EP signal. Such a filter can be used to identify a baseline or average response in the EP signal. A 2-D filter can be used to identify points where the EP signal changes sharply, which may be useful for detecting edges in EP signals. Example 2-D filters detect edges, e.g., by calculating a gradient of the EP signals.

The type of 1st order filter depends on the purpose of the filtering and can be a low-pass filter, a high-pass filter, a band-pass filter and/ or a band-stop filter. A low-pass filter can be used to remove high-frequency noise from the EP signals. A high-pass can be used to eliminate low-frequency drift or baseline wander in the EP signals and/or emphasize high-frequency components that correspond to rapid changes in the EP signals, which allows for detecting spatial variations or edges in EP signals, highlighting areas where there is a significant change in amplitude. A band-pass filter can be used for isolating a particular frequency band of interest in the EP signals. A band-stop filter can be used to remove specific frequency components.

Other analysis tools identify spatial regions with differing EP signals, determine changes in spatial correlation as a function of time, determine whether the EP signals are uniform through the organoid, identify a region that is not responsive to the CUT, and/or identify changes over time. Other analysis tools temporally correlate and/or determine variations in the EP signal as a function of location in the organoid, identify a spatial region whose EP signals vary over time, provide changes in temporal correlation as a function of time, identify particular locations or regions in the organoid whose EP signals are behaving differently over time, and/or identify whether such changes are uniform throughout the organoid. Other analysis tools perform a frequency domain analysis to identify frequency components of EP signals spatially and/or temporally, compare values against values established by the user or against database of expected values obtained from previous studies using the same organoid or CUT, or different organoids or CUTs.

The perfusion information analyzer 304 is configured to automatically control the perfusion system 114 based on user input and/or data from the EP analyzer 302. For example, in one instance this includes controlling the perfusion system 114 where the EP signals are indicative of excess or insufficient perfusion media. In another instance, the perfusion information analyzer 304 provides information for a user to control the perfusion system 114 based on user input and/or data from the EP analyzer 302.

The CUT information analyzer 306 is configured to automatically control the CUT system 118 based on user input and/or data from the EP analyzer 302. For example, in one instance this includes controlling the CUT system 118 where the EP signals are indicative of excess or insufficient CUT. In another instance, the CUT information analyzer 306 provides information for a user to control the CUT system 118 based on user input and/or data from the EP analyzer 302.

The correlation analyzer 308 is configured to determine a correlation with respect to EP signals from organoids with known characteristics. For example, in one instance, the correlation analyzer 308 is configured to compare a current state (from the sensed EP signals) of growing organoid with information indicative of a developed/mature organoid to determine whether the organoid is ready for a study. In another instance, the correlation analyzer 308 is configured to compare a state of an organoid before a CUT and/or stimulation is applied with a state of the organoid after the CUT and/or stimulation is applied. From the comparison, the correlation analyzer 308 can determine a correlation between the states of the organoid before and after the CUT and/or stimulation is applied. In another instance, the correlation analyzer 308 is configured to compare a state of an organoid after a CUT and/or stimulation is applied with information indicative of a "normal" organoid that has not been subject to the CUT and/or stimulation. From the comparison, the correlation analyzer 308 can determine a correlation between the organoid under test and the "normal" organoid.

Additionally, or alternatively, the correlation analyzer 308 is configured to analyze correlations between EP signals and the perfusion information and/or CUT information. For example, in one instance this includes determining spatial and/or temporal correlations in the EP signals with changes in the CUT. In one instance, the correlation analyzer 308 includes artificial intelligence (AI) trained to identify trends in the EP signals as a function of time, spatial location, and/or perfusion information and/or CUT information. Results of the correlation analysis are provided via a display device and/or otherwise, to the EP signal analyzer 302, to the perfusion information analyzer 304, to the CUT information analyzer 306, etc.

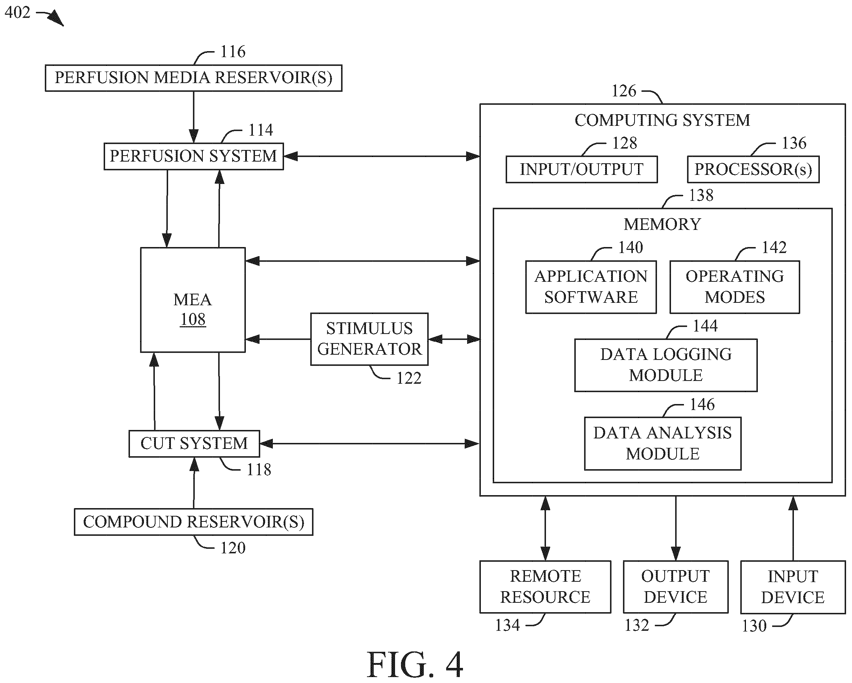

FIGURE 4 illustrates a variation 402 of the system 102 in FIGURE 1. In this example, the variation 402 is substantially similar to the system 102 in FIGURE 1 except that the variation 402 does not include the headstage 104 or the interface board 124. Instead, with the variation 402, the mesh MEA device 108 is in electrical communication with the perfusion system 114, the cut system 118, the stimulus generator 122 and the computing system 126, and does not communicate therewith via the headstage 104 or the interface board 124. The remaining elements function as described in connection with FIGURE 1 and will not be duplicated in the description of FIGURE 4 for sake of clarity and conciseness.

As described herein, the event identifier 310 identifies EP signals that are likely to be of interest to the user. This includes events corresponding to neurological organoids such as those involving brain or other neurological organoids, cardiac or heart organoids, etc., by themselves and/or during and after application of a CUT, stimulation, etc. For neurological signals, events of interest include spike, burst, network, etc. activity. For cardiac (heart) signals, events of interest include the duration of the cardiac field potential, as a representation of the duration of the QT interval, and the spike as a representation of a cardiac beat (from here onwards referred to as a beat). For both neurological and cardiac signals, with respect to a CUT, other events of interest include events at a start time at which a CUT is supplied, associated with a flow at which the CUT is supplied, associated with a concentration at which the CUT is supplied, indicating changes in activity patterns before and after application of the CUT, etc. For both neurological and cardiac signals, with respect to stimulation, other events of interest include events after stimulation is supplied, associated with a spike, indicating changes in activity patterns before and after application of the stimulation, etc.

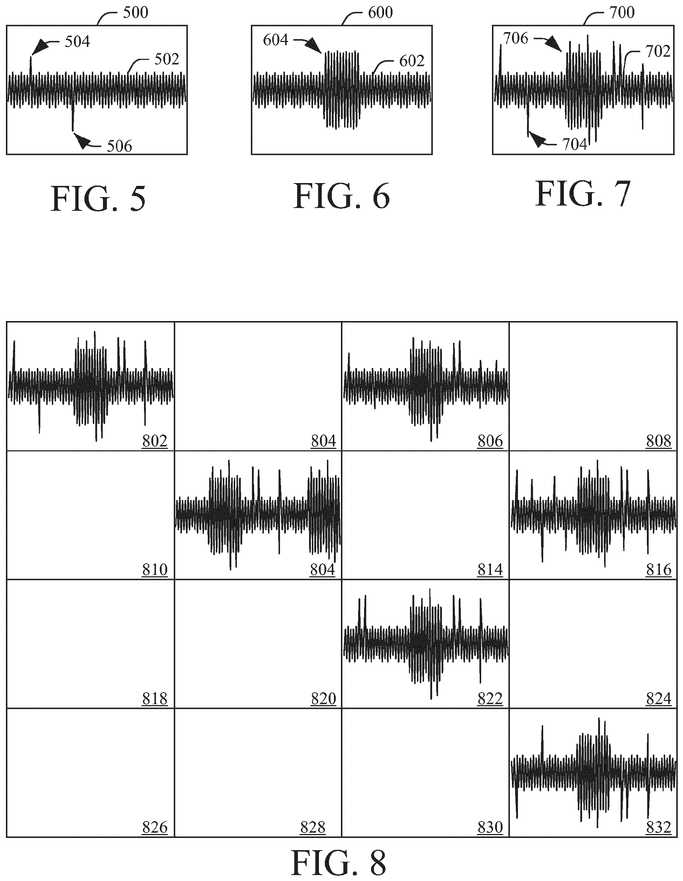

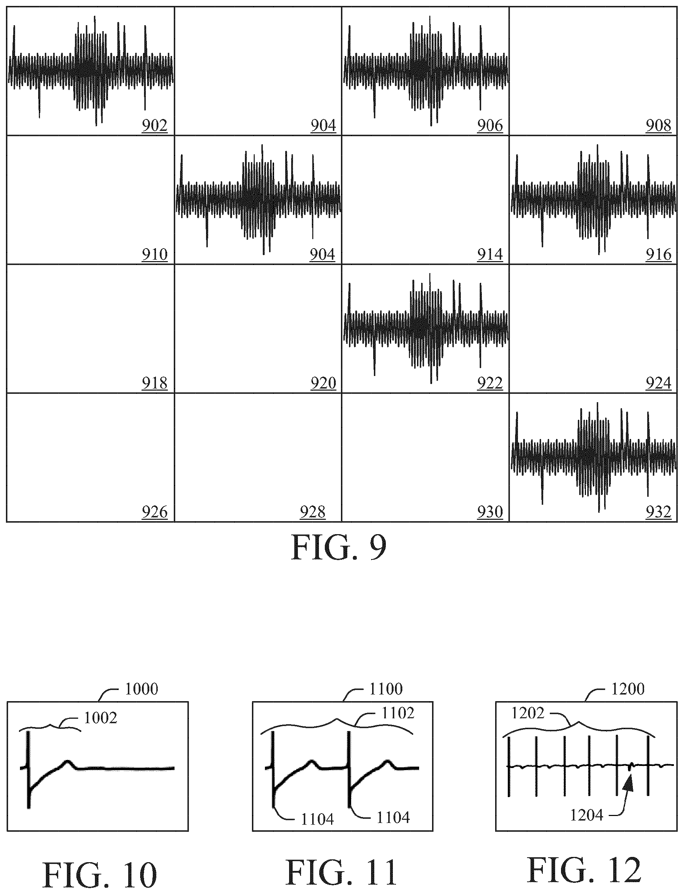

FIGURES 5, 6, 7, 8, 9, 10, 11 and 12 illustrate non-limiting examples of snippets of EP signals with activity of interest corresponding to brain and heart organoids that include events that the event identifier 310 is configured to identify. FIGURES 5, 6, 7, 8 and 9 illustrate snippets of neurological signals, and FIGURES 10, 11 and 12 illustrate snippets of cardiac signals. In general, the EP signals include events that may correspond to "normal" events, "abnormal" events, a particular condition of an organoid, a state of a particular location(s) within the organoid, etc. Examples of normal events include signals indicative of normal cardiac function. Examples of abnormal events include signals indicative of abnormal function such as arrythmias or seizures. Examples of signals indicative of a state of the organoid include signals indicative of the developmental state of the organoid (e.g., developed/mature or not) or necrosis.

Initially referring to FIGURE 5, a window 500 displays a snippet 502 of an EP signal output by a microelectrode for a brain organoid cell. The y-axis represents voltage amplitude and the x-axis represents time. In this example, the event of interest is a spike, and the snippet 502 includes a spike 504 and a spike 506. In one instance, the event identifier 310 detects the spikes 504 and 506 by comparing a voltage amplitude of the snippet 502 with upper and lower thresholds where a spike is detected whenever the voltage amplitude of the signal crosses the upper or lower thresholds. Additionally, or alternatively, the event identifier 310 detects spikes 504 and 506 using a frequency domain approach such as a wavelet transform that decomposes the snippet 502 into different frequency components that are analyzed to identify spikes. In one instance, the event identifier 310 identifies and displays the spike activity to visualize spike spatial patterns, spike activity across different regions, spike intensity of activity across different regions, spike regularity, etc. The event analyzer 312 analyzes the events identified by the event identifier 310 to facilitate determining information such as peak-to-peak voltage amplitude, a slope, spikes per second per electrode, a total number of spikes, the number of microelectrodes with activity greater than a minimum spike rate, etc. The correlation analyzer 308 correlates the information with known characteristics.

Moving to FIGURE 6, a window 600 displays a snippet 602 of an EP signal output by a microelectrode for a brain organoid cell. The y-axis represents voltage amplitude and the x-axis represents time. In this example, the event of interest is a burst, and the snippet 602 includes an electrode burst 604. In general, a burst includes a series or group of spikes within a given time duration. In one instance, the event identifier 310 detects the burst 604 using approaches similar to detecting spikes, including time domain, frequency domain, and mixed time and frequency domain analysis. In one instance, the event identifier 310 identifies and displays the burst activity to visualize burst spatial patterns, burst activity across different regions, burst intensity of activity across different regions, etc. The event analyzer 312 analyzes the events identified by the event identifier 310 to facilitate determining information such as the number of bursts over a given time duration, the duration of an individual burst, the number of spikes in a burst, the time between bursts, etc. The correlation analyzer 308 correlates the information with known characteristics.

Moving to FIGURE 7, a window 700 includes a snippet 702 of an EP signal output by a microelectrode for a brain organoid cell. The y-axis represents voltage amplitude and the x-axis represents time. In this example, there are multiple brain organoid events of interest, including spike activity and burst activity. In this example, the snippet 702 includes spikes (similar to spike in the snippet 502 described in connection with FIGURE 5) such as a spike 704, an electrode burst 706 (similar to the burst in the snippet 602 described in connection with FIGURE 6). The spike 704 and the burst 602 can be visualized, analyzed and/or correlated as described in connection to FIGURES 5 and 6.

The example in FIGURE 8 is a simplified schematic representation of a two-dimensional array of windows, including a window 802, a window 804, a window 806, a window 808, a window 810, a window 812, a window 814, a window 816, a window 818, a window 820, a window 822, a window 824, a window 826, a window 828, a window 830, and a window 832. In this example, the windows 802-832 are used to analyze brain organoid network activity. For example, the windows 802, 806, 812, 816, 822 and 832 correspond to microelectrodes with an output signal indicating organoid cell activity. In one instance, the event identifier 310 identifies and displays the network activity to visualize spike and/or burst activity (e.g., as described above), etc. In this example, the snippets in the windows 802, 806, 812, 816, 822 and 832 show a group of microelectrodes in a given vicinity with burst and spike activity. However, the burst and spike activity is different in windows 802, 806, 812, 816, 822 and 832, indicating that the network is not synchronized. The event analyzer 312 and/or the correlation analyzer 308 analyze the events by comparing and/or correlating the information determined for the snippet in each window with the information determined for the snippets in the other windows, before and/or after a CUT, stimulation, etc.

The example in FIGURE 9 includes a two-dimensional array of windows, including a window 902, a window 904, a window 906, a window 908, a window 910, a window 912, a window 914, a window 916, a window 918, a window 920, a window 922, a window 924, a window 926, a window 928, a window 930, and a window 932. Similar to FIGURE 8, the windows 902-932 are used to analyze brain organoid network synchronicity. The windows 902, 906, 912, 916, 922 and 932 correspond to microelectrodes with an output signal indicating organoid cell activity. Similar to FIGURE 8, the event identifier 310 identifies and displays network activity to visualize spike and/or burst activity (e.g., as described above), etc. However, in this example, the snippets in windows 902, 906, 912, 916, 922 and 932 show similar burst and spike activity, indicating the corresponding microelectrodes are likely part of a same synchronized network (i.e., a connection of microelectrodes). Likewise, the event analyzer 312 and/or the correlation analyzer 308 analyze the events by comparing and/or correlating the information determined for the snippet in each window with the information determined for the snippets in the other windows, before and/or after a CUT, stimulation, etc.

Moving to FIGURE 10, a window 1000 includes an EP signal 1002 output by a microelectrode for a heart organoid cell. The y-axis represents voltage amplitude and the x-axis represents time. In this example, the event of interest is a cardiac field potential. The EP signal 1002 represents a cardiac field potential. In one instance, the event identifier 310 identifies and displays field potentials to visualize activity across microelectrodes, the electrodes detecting activity, etc. The event analyzer 312 analyzes the events and determines information such as a voltage amplitude (e.g., peak-to-peak) of a wave, a duration of the field potential, etc. The correlation analyzer 308 correlates the information with known characteristics.

FIGURE 11 includes a window 1100 that includes an EP signal 1102 output by a microelectrode for a heart organoid cell. The y-axis represents voltage amplitude and the x-axis represents time. In this example, the event of interest is a beat, and the EP signal 1102 includes two beats 1104. In one instance, the event identifier 310 identifies and displays beats to visualize which microelectrodes are detecting a beat, beat spatial patterns across microelectrodes, etc. The event analyzer 312 analyzes the events and determines information such as a number of microelectrodes detecting a beat, the distance between beats, etc. The correlation analyzer 308 correlates the information with known characteristics.

FIGURE 12 includes a window 1200 that includes an EP signal 1202 output by a microelectrode for a heart organoid cell. The y-axis represents voltage amplitude and the x-axis represents time. In this example, the event of interest is an early after depolarization (EAD) abnormal event, and the EP signal 1202 includes an EAD event 1204. In one instance, the event identifier 310 identifies and displays EADs to visualize which electrodes are detecting an EAD, EAD spatial patterns across microelectrodes, etc. The event analyzer 312 analyzes the events and determines information such as a time duration between EADs, the time from an EAD to the T-wave, etc. The correlation analyzer 308 correlates the information with known characteristics.

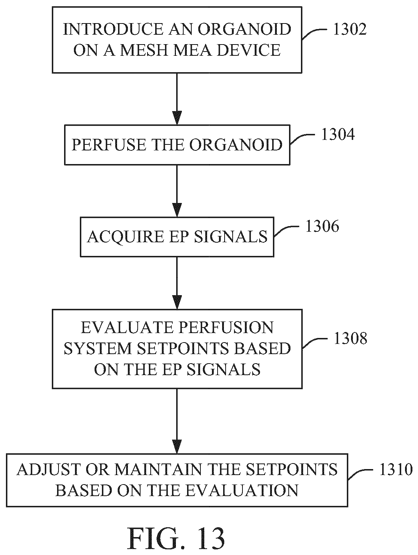

FIGURE 13 illustrates a method in accordance with an embodiment herein. It is to be appreciated that the ordering of the acts of the method is not limiting. As such, other orderings are contemplated herein. In addition, one or more acts may be omitted, and/or one or more additional acts may be included.

At 1302, an organoid of interest is introduced on the mesh MEA device 108, as described herein and/or otherwise. At 1304, perfusion media is provided to the organoid during the longitudinal analysis to promote organoid growth and maintain viability based on a set of predetermined setpoints (e.g., user-defined, AI generated, etc.), as described herein and/or otherwise. At 1306, the data analysis module 146 receives a set of EP signals produced by the organoid from a set of electrodes of the mesh MEA device 108 that are in electrical communication with the organoid, as described herein and/or otherwise. At 1308, the data analysis module 146 determines whether the set of predetermined setpoints are sufficient for the longitudinal analysis based on the set of EP signals, as described herein and/or otherwise. At 1310, the set of predetermined setpoints are maintained or adjusted based on the determination, as described herein and/or otherwise.

For example, where the determination indicates insufficient perfusion media is being supplied to the organoid for the longitudinal analysis, the set of predetermined setpoints is adjusted to supply more perfusion media to the organoid, and where the determination indicates excess perfusion media is being supplied to the organoid for the longitudinal analysis, the set of predetermined setpoints is adjusted to supply less perfusion media to the organoid. In one instance, the adjustment is provided by the data analysis module 146. In another instance, the adjustment is based on a user input. Acts 1304 through 1310 are repeated during the longitudinal study to ensure the organoid remains viable for the duration of the study, as described herein and/or otherwise. The EP signals can also be processed to identify activity events, which can be analyzed and/or correlated as described herein and/or otherwise.

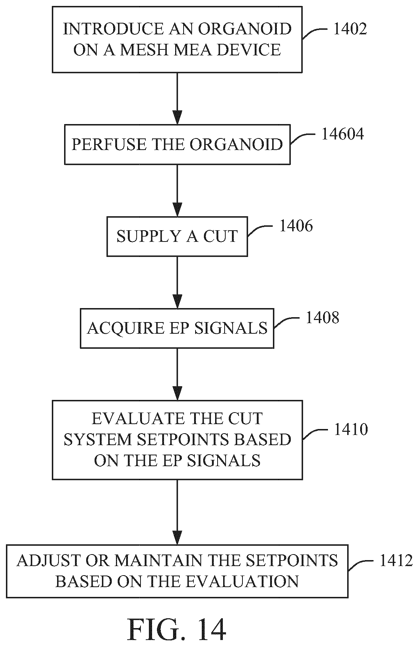

FIGURE 14 illustrates another method in accordance with an embodiment herein. It is to be appreciated that the ordering of the acts of the method is not limiting. As such, other orderings are contemplated herein. In addition, one or more acts may be omitted, and/or one or more additional acts may be included.

At 1402, an organoid of interest is introduced on the mesh MEA device 108, as described herein and/or otherwise. At 1404, perfusion media is provided to the organoid during the longitudinal analysis to promote organoid growth and viability, as described herein and/or otherwise. At 1406, a CUT is provided to the organoid based on a set of predetermined setpoint, as described herein and/or otherwise. At 1408, the data analysis module 146 receives a set of EP signals produced by the organoid from a set of electrodes of the mesh MEA device 108 that are in electrical communication with the organoid, as described herein and/or otherwise. At 1410, the data analysis module 146 determines whether the set of predetermined setpoints are sufficient for the longitudinal analysis based on the set of EP signals, as described herein and/or otherwise. At 1412, the set of predetermined setpoints are maintained or adjusted based on the determination, as described herein and/or otherwise.

For example, where the determination indicates insufficient CUT media is being supplied to the organoid for the longitudinal analysis, the set of predetermined setpoints is adjusted to supply more CUT to the organoid, and where the determination indicates excess CUT is being supplied to the organoid for the longitudinal analysis, the set of predetermined setpoints is adjusted to supply less CUT to the organoid. In one instance, the adjustment is provided by the data analysis module 146. In another instance, the adjustment is based on a user input. Acts 1404 through 1412 are repeated during the study to ensure the organoid remains viable for the duration of the study. The EP signals can also be processed to identify activity events, which can be analyzed and/or correlated as described herein and/or otherwise. The method of FIGURES. 13 and 14 can be combined to monitor and control both the perfusion media (FIGURE 13) and the CUT (FIGURE 14), as described herein and/or otherwise.

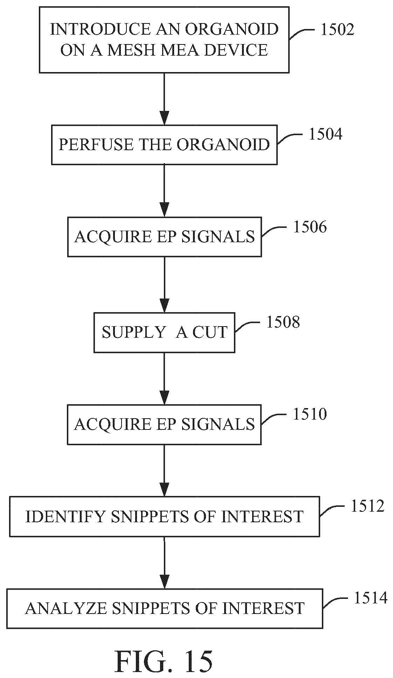

FIG. 15 illustrates another method in accordance with an embodiment herein. It is to be appreciated that the ordering of the acts of the method is not limiting. As such, other orderings are contemplated herein. In addition, one or more acts may be omitted, and/or one or more additional acts may be included.

At 1502, an organoid of interest is introduced on the mesh MEA device 108, as described herein and/or otherwise. At 1504, perfusion media is provided to the organoid during the longitudinal analysis to promote organoid growth and viability, as described herein and/or otherwise. At 1506, the data analysis module 146 receives a set of EP signals produced by the organoid from a set of electrodes of the mesh MEA device 108 that are in electrical communication with the organoid, as described herein and/or otherwise. At 1508, a CUT is provided to the organoid based on a set of predetermined setpoints, as described herein and/or otherwise. At 1510, the data analysis module 146 receives a set of EP signals produced by the organoid from the set of electrodes of the mesh MEA device 108 that are in electrical communication with the organoid, as described herein and/or otherwise. In one instance, it is not feasible or practical for a user to analyze the large volume of EP signals.

At 1512, the event identifier 310 of the data analysis module 146 determines snippets of the EP signals from the longitudinal study that are likely to be of interest for analysis based on predetermined criteria, as described herein and/or otherwise. In one instance, this reduces the volume of EP signals for analysis to a level that is feasible and practical for a user. At 1514, a summary of the identified events is provided, as described herein and/or otherwise. The event analyzer 312 of the data analysis module 146 is utilized to analyze one or more of the identified snippets in the summary, as described herein and/or otherwise. The event analyzer 312 can also be utilized to analyze one or more of the snippets of the EP signals that are not in the summary. The correlation analyzer 308 can determine correlations amongst EP signals, perfusion information, and/or CUT information, including determining spatial and/or temporal correlations in the EP signals with changes in the CUT.

The above methods can be implemented at least in part by way of computer readable instructions, encoded, or embedded on the computer readable storage medium, which, when executed by a computer processor, cause the processor to carry out the described acts or functions. Additionally, or alternatively, at least one of the computer readable instructions is carried out by a signal, carrier wave or other transitory medium, which is not computer readable storage medium.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

The word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to an advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (102) for longitudinal analysis of an organoid, comprising:
a region (106) configured to support a Microelectrode Array (MEA) device (108) carrying an organoid for longitudinal analysis;
wherein the MEA device (108) includes a MEA supporting the organoid, a well configured to hold perfusion media for the organoid, a port for supplying the perfusion media to the well, and nodes in the MEA for sensing electrophysiological (EP) signals produced by the organoid from an interior of the organoid;
a perfusion media interface (124) to a perfusion system (114); and
a computing system (126) configured to identify events in the EP signals for analysis based on predetermined criteria, wherein the identified events include less than an entirety of each of the EP signals, provide a summary of the identified events, and analyze a user selected set of the identified events in the summary.

2. The system (102) of claim 1, wherein a summary for an identified event includes an identification of a microelectrode of the MEA sensing the event and a time stamp.

3. The system (102) of claim 2, wherein the computing system (126) presents the summary as links that automatically display an event in response to user selection of the event via a corresponding link.

4. The system (102) of claim 3, wherein the computing system (126) is configured to process a displayed event based on a user input.

5. The system (102) of claim 1, further comprising:
a perfusion system (114) configured to supply the perfusion media to the MEA device through the perfusion media interface.

6. The system (102) of claim 5, wherein the computing system (126) is configured to control the perfusion system (114) based on an analysis of the EP signals.

7. The system (102) of claim 5, wherein the computing system (126) is configured to automatically change a setpoint of the perfusion system (114) based on the analysis of the EP signals.

8. The system (102) of claim 5, wherein the well is configured to concurrently hold a compound under test (CUT), and further comprising:
a CUT interface; and
a CUT system (118) configured to supply the CUT to the MEA device (108) through the CUT interface.

9. The system (102) of claim 8, wherein the computing system (126) is configured to control the CUT system (118) based on the analysis of the EP signals.

10. The system (102) of claim 8, wherein the computing system (126) is configured to automatically change a setpoint of the CUT system (118) based on the analysis of the EP signals.

11. The system (102) of claim 1, wherein the MEA device is a mesh MEA device (108).

12. A method for longitudinal analysis of an organoid, comprising:
Introducing (1302) an organoid to a MEA device (108);
supplying (1304) perfusion media to the MEA device (108) to maintain organoid viability for the longitudinal analysis;
sensing (1306) EP signals produced by the organoid from an inside of the organoid with nodes of the MEA device (108); and
processing (1308) the EP signals with a computing system (126), wherein the processing includes identifying events for analysis in the EP signals based on predetermined criteria, providing a summary of the identified events, and analyzing a user selected set of the identified events in the summary.

13. The method of claim 12, wherein the organoid is a neurological or cardiac organoid, and the identifying includes identifying electrical activity events of the organoid.

14. The method of claim 13, wherein the electrical activity events for the neurological organoid include one or more of spike, burst, or network activity events, and the electrical activity events for the cardiac organoid include one or more cardiac field potential or beat events.

15. The method of claim 13, wherein the analyzing includes determining information indicative of a functioning of the organoid based on the identified electrical activity events.
